# EUROPÄISCHE PATENTSCHRIFT

(11) **EP 3 222 173 B1**
(45) Veröffentlichungstag und Bekanntmachung des Hinweises auf die Patenterteilung: **26.06.2019**
(21) Anmeldenummer: 17159637.2
(22) Anmeldetag: 07.03.2017
(51) Int. Cl.: A47J 19/02, A47J 43/042

(54) **KÜCHENMASCHINE**
FOOD PROCESSOR
ROBOT MENAGER

(30) Priorität: 25.03.2016 AT 502482016
(43) Veröffentlichungstag der Anmeldung: 27.09.2017
(73) Patentinhaber: Innerhuber, Johann, 4441 Behamberg (AT)
(72) Erfinder: Innerhuber, Johann, 4441 Behamberg (AT)
(74) Vertreter: Patentanwaltskanzlei Hübscher

(56) Entgegenhaltungen:
- DE-A1-102009 006 672
- US-A- 2 315 018

## Beschreibung

Die Erfindung bezieht sich auf eine Küchenmaschine mit einer Antriebseinheit auf die ein bodenseitig mit einem drehangetriebenen Rotor versehener Mixbehälter aufgesetzt ist, wobei einem Deckel des Mixbehälters ein Zitrusfrüchtepresskopf zugeordnet ist, der in eine Einfüllöffnung des Deckels eingesetzt ist, wobei der Zitrusfrüchtepresskopf über den Zitrusfrüchtepresskopfboden hinaus mit einer Welle verlängert ist, die in einem, in die Einfüllöffnung eingesetzten, Lagerstopfen drehbar gelagert ist, der wenigstens eine Durchlassöffnung aufweist und die über eine Kupplung mit dem Rotor drehantriebsverbunden ist.

Eine derartige Küchenmaschine offenbart beispielsweise die US 2 315 018 A. Weitere Vorrichtungen zum Entsaften von Zitrusfrüchten zeigen beispielsweise die US 4 744 522 A und die US 2 295 922 A. Eine gattungsgemäße Vorrichtung zeigt die DE 10 2009 006672 A1, gemäß der als Kupplung eine Überlastkupplung vorgesehen ist, die bei Überlast dadurch auskuppelt, dass ein stabartiger Kupplungsfortsatz ausfedert.

Ein Zubehör für eine Küchenmaschine mit einer Kombination aus Zitruspresse und einer Vorrichtung zum Trennen von Eiweiß und Dotter eines rohen Eies ist aus der EP 1 917 889 A1 bekannt. Das Zubehör ist verdrehgesichert in eine Einfüllöffnung eines Standmixerdeckels einsetzbar. Der Standmixer umfasst unter anderem eine Antriebseinheit auf die der bodenseitig mit Mixflüqeln, die Teil des Rotors sind, versehene Mixbehälter aufsetzbar ist. Bei ordnungsgemäß auf die Antriebseinheit aufgesetztem Mixbehälter sind die Mixflügel mit der Antriebseinheit über eine Kupplung drehantriebsverbunden. Die Rotordrehzahl kann üblicherweise in Stufen oder Stufenlos eingestellt werden. Mit auf den Mixbehälter aufgesetztem und mit dem Zubehör ausgestattetem Deckel können Zitrusfrüchte manuell ausgepresst und kann der gewonnene Saft zugleich in den Mixbehälter übergeleitet werden.

Die DE 1084457 B offenbart einen Deckel mit einer trichterförmigen, mit einem Stopfen verschließbaren, Einfüllöffnung für einen mit Mixflüqeln versehenen Mixbehälter. Dabei ist der in die Einfüllöffnung verdrehgesichert eingesetzte Stopfen als Zitrusfüchtepresskopf ausgebildet, der in seiner unteren Mantelfläche im Bereich der Einfüllöffnung Abflussrinnen für den ausgepressten Saft aufweist. Auch mit dieser Vorrichtung können bei in den Deckel eingesetztem Stopfen Zitrusfrüchte manuell ausgepresst werden, wobei der ausgepresste Saft durch die Abflussrinnen zwischen Abflußstutzen und Stopfen in den Mixbecher abfließen kann.

Aus der WO 2013 079 444 A1 ist ein Aufsatz für eine Schüssel einer Küchenmaschine bekannt, der mit einem elektrischen Antrieb zum Antreiben eines Werkzeugs der Küchenmaschine ausgestattet ist. Der Aufsatz umfasst eine Öffnung für ein Einfüllen von Nahrungsmitteln in die Schüssel, womit während des Betriebs jederzeit Nahrungsmittel durch den Aufsatz in die Schüssel aufgegeben werden können, auf die der Aufsatz aufgesetzt ist. Ein Werkzeug mit dem Nahrungsmittel verarbeitet werden kann Bestandteil eines Anbauteils, beispielsweise eine Zitruspresse, sein, welche während des Betriebs auf den Aufsatz aufgesetzt ist. Ausgepresster Saft kann durch die Öffnung des Aufsatzes hindurch in die Schüssel gelangen. Alternativ oder ergänzend kann zudem ein Werkzeug unterhalb des Aufsatzes angebracht sein. Eine derartige Zitruspresse ist auch aus der US 6070519 A bekannt.

Der Erfindung liegt somit die Aufgabe zugrunde, einen Mixer der eingangs geschilderten Art, der einen Deckel aufweist, der bei Bedarf auch mit einem herkömmlichen Stopfen dicht verschließbar ist, mit einfachen Mitteln derart weiterzubilden, dass damit ein vorteihaftes Auspressen von Zitrusfrüchten möglich ist.

Die Erfindung löst die gestellte Aufgabe dadurch, dass die Kupplung eine Klauenkupplung ist, deren Klauen mit dem Rotor, zwischen dem Rotor zugehörigen Mixflügeln, im Eingriff stehen und dass auf den Deckel ein Fruchtfleisch und Kerne von der Durchlassöffnung fernhaltendes Rückhaltesieb und ein den Zitrusfrüchtepresskopf radial umfassender, zusammen mit dem Rückhaltesieb radial an einer Schulter an der Deckeloberfläche des Deckels gesicherter, Spritzschutztrichter aufgesetzt sind.

Erfindungsgemäß ist der Zitrusfrüchtepresskopf über den Rotor drehangetrieben, sodass für den Zitrusfrüchtepresskopf kein gesonderter Drehantrieb vorgesehen werden muss. Dazu ist der Zitrusfrüchtepresskopf über den Zitrusfrüchtepresskopfboden hinaus mit einer Welle verlängert, welche Welle lösbar mit dem Zitrusfrüchtepresskopf verbunden ist, und die über eine Kupplung drehantriebsfest am Rotor angreift. Um dabei eine ordnungsgemäße Führung der Welle im Bereich des Zitrusfrüchtepresskopfes gewährleisten zu können, ist die Welle im Nahbereich des Zitrusfrüchtepresskopfes mit einem Lager in Form eines Lagerstopfens ausgestattet. Dieser Lagerstopfen ist in den Deckel des Mixbehälters eingesetzt und nimmt die Welle frei drehbar auf. Dieser Lagerstopfen kann ein einfaches Kunststoffgleitlager, ein Kugellager oder ein beliebiges anderes Lager sein oder aufweisen.

Um die Welle möglichst einfach und sicher mit dem Rotor drehantriebsfest verbinden zu können, ist die Kupplung eine Klauenkupplung, deren Klauen mit dem Rotor, zwischen dem Rotor zugehörigen Mixflügeln, im Eingriff stehen. Stehen die Klauen mit dem Rotor dabei mittels einer Rast in Richtung der Drehachse gesichert im Eingriff, so ist ein unerwünschtes Abziehen bzw. abwerfen der Welle vom Rotor im Pressbetrieb sicher vermieden, da dies gegebenenfalls eine Zerstörung der Welle zur Folge hätte.

Zudem ist dem Deckel ein Rückhaltesieb zugeordnet, welches Fruchtfleisch und Kerne von der Durchlassöffnung fernhält. Um beim Auspressen von Zitrusfrüchten ein Abspritzen in radialer Richtung von Fruchtsaft zu vermeiden, ist dem Deckel ein den Zitrusfrüchtepresskopf radial umfassender, radial gesicherter Spritzschutzring, insbesondere Spritzschutztrichter, zugeordnet. Dieser ist zusammen mit dem Rückhaltesieb radial gesichert auf den Deckel aufgesetzt. Die radiale Sicherung übernimmt eine an der Deckeloberfläche vorgesehene umlaufende Schulter. Eine vom Deckel nach unten abragende Schulter hingegegen sorgt dafür, dass der Deckel am Mixbehälter gesichert ist.

Um einen Deckel zu haben, der bei Bedarf auch mit einem herkömmlichen Stopfen dicht verschließbar ist, kann der Lagerstopfen wenigstens eine Durchlassöffnung aufweisen, der gegebenenfalls ein Rückhaltesieb zugeordnet ist. Der Zitrusfrüchtesaft kann dann durch diese Durchlassöffnung im Lagerstopfen von Deckeloberseite in den Mixbehälter einfließen. Etwaige Kerne oder etwaiges Fruchtfleisch können mit einem Rückhaltesieb zurückgehalten werden. Besonders einfache und elegante Konstruktionsverhältnisse ergeben sich dabei, wenn der Lagerstopfen wenigstens eine von seinem Außenumfang zur Drehachse hin zurückspringende, die Durchlassöffnung freigebende Einbuchtung aufweist. Diese Konstruktion lässt sich bei vom Deckel abgenommenem Lagerstopfen besonders einfach reinigen.

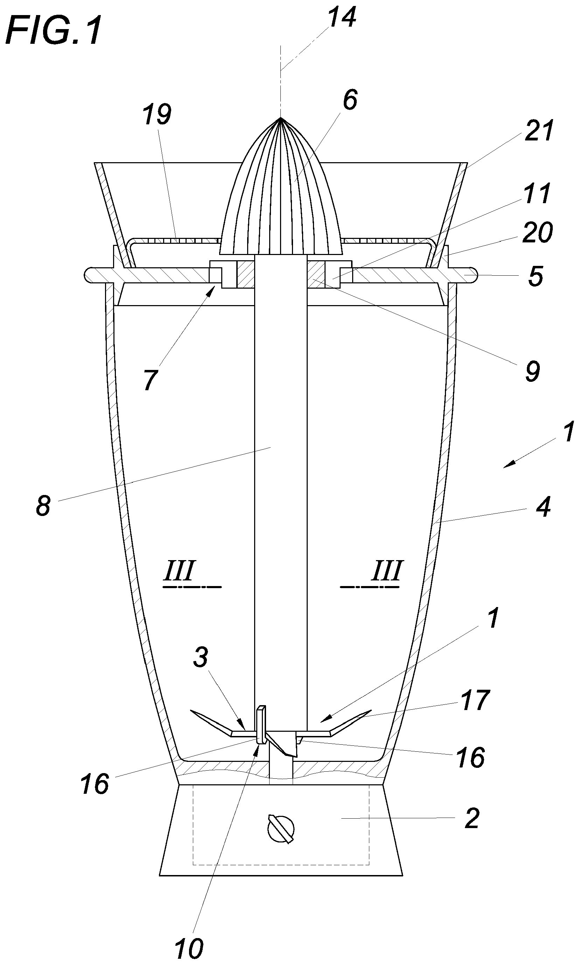

In der Zeichnung ist der Erfindungsgegenstand beispielsweise dargestellt. Es zeigen
- Fig. 1: eine erfindungsgemäße Küchenmaschine im teilgeschnittenen Querschnitt,
- Fig. 2: den vergrößerten Lagerstopfen aus Fig. 1 in vergrößerter Draufsicht,
- Fig. 3: ein Vergrößerter Ausschnitt der Küchenmaschine im Schnitt nach der Linie III-III aus Fig. 1,
- Fig. 4: eine teilgeschnittene Seitenansicht des Mixflügels aus Fig. 3 im Schnitt nach der Linie IV-IV aus Fig. 3 und
- Fig. 5: einen alternativen Lagerstopfen mit Rückhaltesieb in Draufsicht.

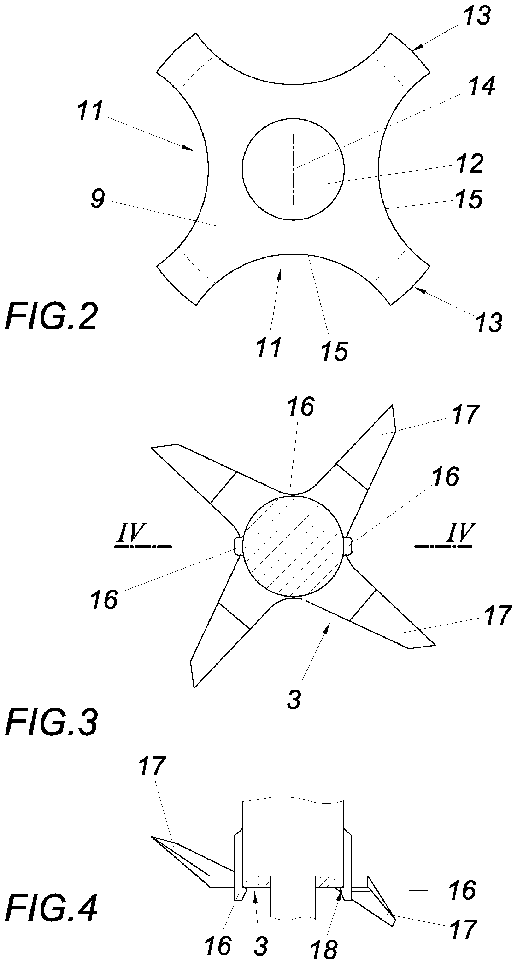

Eine Küchenmaschine 1 umfasst unter anderem eine Antriebseinheit 2, auf die ein bodenseitig mit einem drehangetriebenen Rotor 3 versehener Mixbehälter aufgesetzt ist. Einem Deckel 5 des Mixbehälters 4 ist ein Zitrusfrüchtepresskopf 6 zugeordnet, der in eine Einfüllöffnung 7 des Deckels 5 eingesetzt. Um für einen einfachen Drehantrieb des Zitrusfrüchtepresskopfes 6 zu sorgen, ist dem Zitrusfrüchtepresskopf 6 bodenseitig eine Welle 8 zugeordnet, die in einem, in die kreisrunde Einfüllöffnung 7 eingesetzten, Lagerstopfen 9 drehbar gelagert ist und die über eine Kupplung 10 mit dem Rotor 3 drehantriebsverbunden ist. Der Lagerstopfen 9 weist Durchlassöffnungen 11 für ausgepressten Saft auf. Eine Draufsicht auf einen derartigen Lagerstopfen ist in der Fig. 2 ersichtlich, wobei das zentrale Loch 12 die Welle 8 aufnimmt und bei entsprechender Werkstoffpaarung ein Gleitlager bildet sowie der Lagerstopfen 9 vier von seinem Außenumfang 13 zur Drehachse 14 hin zurückspringende, die Durchlassöffnungen 11 freigebende Einbuchtungen 15 aufweist.

Die Kupplung ist im dargestellten Ausführungsbeispiel eine Klauenkupplung, deren Klauen 16 zwischen dem Rotor 3 zugehörigen Mixflügeln 17 mit dem Rotor 3 im Eingriff stehen, wobei die Klauen 16 mit einer Rast 18 in Richtung der Drehachse 14 gegen ein Abziehen gesichert mit dem Rotor 3 im Eingriff stehen. Zum Abziehen muss die Rastkraft bewusst überwunden werden.

Dem Deckel 5 ist ein Rückhaltesieb 19 zugeordnet, mit dem Fruchtfleisch und Kerne von den Durchlassöffnungen 11 ferngehalten werden können. Zudem gehört dem Deckel 5 ein den Zitrusfrüchtepresskopf 6 umfassender, radial an einer Schulter 20 des Deckels 5 gesicherter Spritzschutztrichter 21 zu.

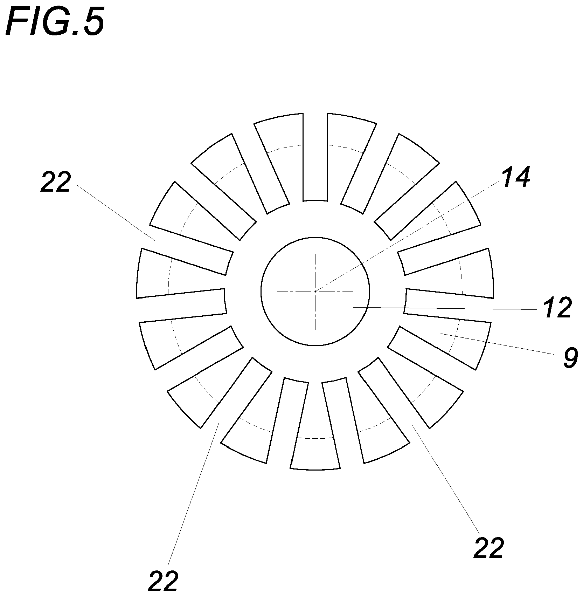

Fig. 5 zeigt einen alternativen Lagerstopfen 9 mit Rückhaltesieb 22 in Draufsicht. Das Rückhaltesieb 22 ist Teil des Lagerstopfens 9 und wird von radialen Schlitzen gebildet.

## Patentansprüche

1. Küchenmaschine (1) mit einer Antriebseinheit (2) auf die ein bodenseitig mit einem drehangetriebenen Rotor (3) versehener Mixbehälter (4) aufgesetzt ist, wobei einem Deckel (5) des Mixbehälters (4) ein Zitrusfrüchtepresskopf (6) zugeordnet ist, der in eine Einfüllöffnung (7) des Deckels (5) eingesetzt ist, wobei der Zitrusfrüchtepresskopf (6) über den Zitrusfrüchtepresskopfboden hinaus mit einer Welle (8) verlängert ist, die in einem, in die Einfüllöffnung (7) eingesetzten, Lagerstopfen (9) drehbar gelagert ist, der wenigstens eine Durchlassöffnung (11) aufweist und die über eine Kupplung (10) mit dem Rotor (3) drehantriebsverbunden ist, **dadurch gekennzeichnet, dass** die Kupplung (10) eine Klauenkupplung ist, deren Klauen (16) mit dem Rotor (3), zwischen dem Rotor (3) zugehörigen Mixflügeln (17), im Eingriff stehen und dass auf den Deckel (5) ein Fruchtfleisch und Kerne von der Durchlassöffnung (11) fernhaltendes Rückhaltesieb (19) und ein den Zitrusfrüchtepresskopf (6) radial umfassender, zusammen mit dem Rückhaltesieb (19) radial an einer Schulter (20) an der Deckeloberfläche des Deckels (5) gesicherter, Spritzschutztrichter (21) aufgesetzt sind.

2. Küchenmaschine (1) nach Anspruch 1, **dadurch gekennzeichnet, dass** der Lagerstopfen (9) wenigstens eine von seinem Außenumfang (13) zur Drehachse (14) hin zurückspringende die Durchlassöffnung (11) freigebende Einbuchtung (15) aufweist.

3. Küchenmaschine (1) nach Anspruch 1 oder 2, **dadurch gekennzeichnet, dass** die Klauen (16) mit dem Rotor (3) mittels einer Rast (18) in Richtung der Drehachse (14) gesichert im Eingriff stehen.

## Claims

1. Food processor (1) having a drive unit (2) on which a mixing container (4) provided on the bottom with a rotationally driven rotor (3) is placed, wherein a citrus fruit pressing head (6) is allocated to a cover (5) of the mixing container (4) and is inserted into a filling opening (7) of the cover (5), wherein the citrus fruit pressing head (6) is extended beyond the base of the citrus fruit pressing head by a shaft (8) which is rotatably mounted in a bearing stopper (9) inserted into the filling opening (7), said bearing stopper comprising at least one through opening (11), and is connected for rotational driving to the rotor (3) via a coupling (10), **characterised in that** the coupling (10) is a claw coupling, the claws (16) of which are engaged with the rotor (3) between mixing blades (17) appertaing to the rotor (3), and that a retaining screen (19) - which keeps fruit flesh and stones away from the through opening (11) - and a spray guard cone (21) - which radially surrounds the citrus fruit pressing head (6) and is secured, together with the retaining screen (19), radially on a shoulder (20) on the cover surface of the cover (5) - are placed onto the cover (5).

2. Food processor (1) as claimed in claim 1, **characterised in that** the bearing stopper (9) comprises at least one concave area (15) recessed from the outer periphery (13) thereof towards the axis of rotation (14) and freeing the through opening (11).

3. Food processor (1) as claimed in claim 1 or 2, **characterised in that** the claws (16) are engaged with the rotor (3) by means of a catch (18), being secured in the direction of the axis of rotation (14).

## Revendications

1. Robot ménager (1) avec une unité d'entraînement (2), sur laquelle est posé du côté du fond un bol mélangeur (4) prévu avec un rotor (3) entraîné en rotation, une tête presse agrumes (6) étant associée à un couvercle (5) du bol mélangeur (4), qui est insérée dans une ouverture de remplissage (7) du couvercle (5), la tête presse agrumes (6) étant allongée à travers le fond de tête presse agrumes vers l'extérieur avec un arbre (8), qui est positionné rotatif dans un tampon de palier (9) inséré dans l'ouverture de remplissage (7), qui comporte au moins une ouverture de passage (11) et qui est reliée en entraînement rotatif à travers un accouplement (10) avec le rotor (3), **caractérisé en ce que** l'accouplement (10) est un embrayage à crabots, dont les crabots (16) sont en prise avec le rotor (3), entre les ailes de mélange (17) associées au rotor (3), et **en ce que** sur le couvercle (5) sont posés un tamis de retenue (19) tenant la pulpe et les pépins éloignés de l'ouverture de passage (11) et un entonnoir de protection contre les projections (21) englobant radialement la tête presse agrumes (6), sécurisé radialement sur un épaulement (20) sur la surface supérieure du couvercle (5) ensemble avec le tamis de retenue (19).

2. Robot ménager (1) selon la revendication 1, **caractérisé en ce que** le tampon de palier (9) comporte au moins une échancrure (15) libérant l'ouverture de passage (11), en retrait vers l'axe de rotation (14), de sa périphérie extérieure (13).

3. Robot ménager (1) selon la revendication 1 ou 2, **caractérisé en ce que** les crabots (16) sont en prise de façon sécurisée en direction de l'axe de rotation (14) avec le rotor (3) au moyen d'un dispositif d'enclenchement (18).
